(19) 

Europäisches
Patentamt

European
Patent Office

Office européen
des brevets

(11)  **EP 2 690 603 B1**

(12)  **EUROPEAN PATENT SPECIFICATION**

(45) Date of publication and mention
of the grant of the patent:
**13.04.2016 Bulletin 2016/15**

(21) Application number: **13005023.0**

(22) Date of filing: **10.11.2009**

(51) Int Cl.:
*G07C 5/08* (2006.01)          *A61B 5/18* (2006.01)
*B60T 13/26* (2006.01)        *B60T 13/66* (2006.01)
*B60T 17/18* (2006.01)        *B60T 17/22* (2006.01)
*G07C 5/00* (2006.01)          *G09B 19/16* (2006.01)
*B60T 7/08* (2006.01)          *B60T 7/04* (2006.01)
*B60T 7/22* (2006.01)          *B60W 40/06* (2012.01)
*B60T 7/14* (2006.01)          *G09B 9/04* (2006.01)

(54) **Braking anticipation ability determining system**

System zur Bestimmung der Fähigkeit zu einer Bremsungsantizipation

Système de détermination de capacité d'anticipation de freinage

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **21.11.2008 SE 0850089**

(43) Date of publication of application:
**29.01.2014 Bulletin 2014/05**

(62) Document number(s) of the earlier application(s) in
accordance with Art. 76 EPC:
**09827824.5 / 2 359 344**

(73) Proprietor: **Scania CV AB (publ)**
**151 87 Södertälje (SE)**

(72) Inventors:
• **Andersson, Jonny**
**S-151 68 Södertälje (SE)**

• **Bredberg, Linus**
**S-151 46 Södertälje (SE)**
• **Andersson, Jon**
**S-151 46 Södertälje (SE)**

(74) Representative: **Thum, Bernhard**
**Wuesthoff & Wuesthoff**
**Patentanwälte PartG mbB**
**Schweigerstraße 2**
**81541 München (DE)**

(56) References cited:
WO-A1-00/07150          WO-A1-03/020562
WO-A1-2006/008731     WO-A1-2007/139489
WO-A1-2007/139493     WO-A1-2007/139494
WO-A2-02/33529          DE-A1- 4 401 416
DE-C1- 19 860 248       FR-A1- 2 863 090
US-A- 6 092 021          US-A1- 2008 255 722

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

Field of the invention

The present invention relates to a braking anticipation ability determining system according to the preamble of the independent claim.

Background of the invention

When driving heavy vehicles, such as trucks, buses and the like, the vehicle economy has become an important factor when trying to reduce costs in the business in which the vehicle is used. Following the acquiring cost of the vehicle, the largest items of expenditure for a vehicle consist of fuel costs and service costs. These costs are often connected, i.e. a vehicle that is heavily used both consumes more fuel and is exposed to greater wear with increasing service costs as a consequence.

An existing problem for the companies that use heavy vehicles in their business is nevertheless that it is difficult to establish how great part of the fuel consumption and wear that e.g. is derived from incautious driving, and how great amount that is derived from unfavorable traffic surroundings, such as extremely undulated ground and/or urban environment.

The wear a vehicle is exposed to during incautious driving can at least partially be due to the vehicle being exposed to an excessive amount of unnecessary accelerations and retardations. There is thus a need to evaluate the driving behavior of a driver and try to improve how the driver interacts with the vehicle.

To determine a driver's anticipation ability, a device like the one disclosed in the International application WO 2007/139493 can be used. Braking measures are here taken to determine a difference parameter value that is compared with a reference parameter value. Based on the comparison the driver's anticipation ability may be determined and e.g. shown to the driver on a display in the shape of a percentage between 0 and 100. However, no evaluation of the measures is made.

The patent application WO 2007/133987 discloses a system and method for identifying driving non-events. A driving event can be a non-event if it involves instances beyond a driver's control such as driving over a pothole on the road, a railroad crossing etc. This system and method is thus intended for a passenger car and does not include identifying more complex events related to a heavy vehicle.

Document WO 2007/13949 A1 discloses a device for determining the ability of the driver of a vehicle to choose a brake system. For doing so, the system comprises means for deriving a value representing the usage of each brake system and means for receiving a parameter representing the total usage of a plurality of brake systems, and thus by comparison enabling to assess the ability of the driver to use the brake systems.

Document WO 02/33529 A2 discloses a method of informing a vehicle operator to improve the operator's performance. For doing so, an operator's cognitive load is estimated based on vehicle operating and environment data and vehicle information to be presented to the driver are prioritized based on said cognitive load.

Document US 6 092 021 A discloses a fuel efficiency monitoring and display system which dynamically evaluates vehicle performance parameters to detect conditions that cause excessive fuel consumption

Document WO 00/07150 A1 discloses a system of advising the driver of a motor vehicle as regards the efficiency of his driving style while driving. For doing so, it is evaluated to which degree an actual driving style deviates from a normative one and corresponding information regarding the efficiency are presented to the driver.

Document WO 03/020562 A1 discloses a system in which an actual braking action provided by a supplementary brake is compared to a desired braking action and in case a difference exists, said difference is eliminated.

Document DE 198 60 248 C1 discloses a system for identifying a driver and classifying his driving style such that the vehicle parameters can be adapted in advance.

Document US 2008/255722 A discloses a method for evaluating a driver performance wherein the vehicle operations are detected and compared to predetermined criteria and a grade is calculated based on the respective comparison.

The object of the present invention is to obtain an improved analysis of the behavior of the driver when driving the vehicle, and subsequently help the driver to improve his/her anticipation ability in the traffic, which is important from both a road safety point of view as well as for the total economy of the vehicle.

Summary of the invention

The above-mentioned object is achieved by a braking anticipation ability determining system for a vehicle, comprising an evaluation means to evaluate and grade a driver's braking anticipation ability and a retarder sensing means for sensing a retarder status value representing the status of the retarder as being in an inactivate state, wherein if said retarder status value is in its active state and if the driver gives gas, then feedback is given to the driver to release the retarder.

Preferred embodiments are set forth in the dependent claims.

An auxiliary brake is a supplement to the ordinary wheel brakes of the vehicle and is configured to produce a retarding torque for decreasing the rotational speed of the engine crankshaft. An auxiliary brake may for instance be an engine brake, an exhaust brake, an electromagnetic retarder or a hydraulic retarder. The auxiliary brakes are with advantage used for constant speed keeping purposes in downhill slopes and for moderate decelerations, whereas the vehicle's ordinary wheel brakes are mainly used for powerful and sudden braking operations. With the use of auxiliary brakes, the ordinary wheel brakes, which normally constitute friction brakes of disc brake or drum brake type, are prevented from overheating with the associated risk of brake failure and the wear thereof is reduced.

A driver's braking measure may consist of use of e.g. the service brake (wheel brake) or any use of an auxiliary brake, e.g. retarder, engine brake or exhaust brake, or a combination of the mentioned use of brakes (e.g. constant-speed brake).

Short description of the appended drawings

Figure 1 schematically illustrates a control system for a vehicle, where the present invention is implemented.

Figure 2 illustrates an exemplary method according to the invention.

Detailed description of preferred embodiments of the invention

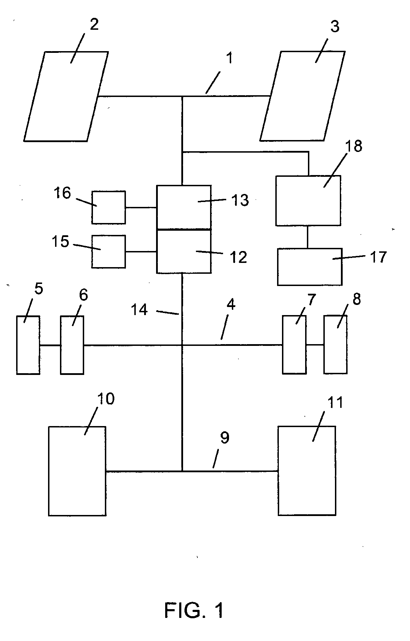

In Fig. 1 is schematically illustrated a control system for a vehicle with which the present invention can be utilized. The vehicle comprises a front shaft 1 with steering wheels 2, 3, a rear drive shaft 4 with driving wheels 5-8, and optionally a rear shaft 9 with wheels 10, 11. Furthermore, the vehicle contains an engine 13 connected to a gear box 12, which drives the drive shaft 4 by means of an output shaft 14 from the gear box. Gear box 12 and motor 13 are controlled by control units 15, 16, respectively, which are controlled by a main control unit 17. The Engine Management System (EMS) 16 controls the motor functions of the vehicle, which, for example, can consist of fuel injection and engine-brake. The control is based on a number of input signals, which can consist of signals from (not shown) throttle controls (the position of the accelerator pedal), speed sensor and brake management system. The Gearbox Management System (GMS) 15 controls the gear functions, wherein, when using an automatic gearbox, the gear shifting can be controlled based on an input signal from speed sensors, in manual gear shifting the shifting can be controlled from an input signal from a gear selector (gear shift lever). Furthermore, the vehicle contains a Brake Management System (BMS) with a brake control unit 17, which controls the brake functions of the vehicle, such as automatic calculation of the load so that a given pedal position always can result in the same brake effect regardless of the load. The brake control unit controls the various brake systems of the vehicle, e.g. retarder and other auxiliary brake systems, exhaust brake and service brake, and sends control signals to system modules (not shown) dispersed on the chassis, where electrical control signals are used, e.g. to adjust brake pressure.

The above described control units constitute mere examples of what can exist in a vehicle. As is appreciated by a person skilled in the art, two or more of the above described control units can, of course, be integrated in one single control unit.

In a vehicle of the kind shown in Fig. 1, it is, as was mentioned above, highly desirable that the driver, when driving the vehicle, is as foresighted as possible to avoid unnecessary braking actions and accelerations, for example those that have been caused by keeping too short distance to the vehicle ahead, or that the driver requests an acceleration on the top of a hill to then immediately brake in a subsequent downhill slope. It is considerably better, in a wear point of view, as well as from a fuel consumption point of view, if the driver as far as possible takes advantage of energy stored in the vehicle, and do not consume it by using the brakes unnecessarily. The ideal vehicle management by the driver at different situations, however, varies to a large extent, and it is today very difficult to find out whether the driver's behavior is good or poor, and, therefore, it is also difficult to give a driver feedback on what can be improved, at the same time as it is difficult for a vehicle owner to assess whether the vehicle is driven in a satisfactory, i.e. economical, fashion.

The international application WO 2007/139493 describes a use of a device for determining a driver's anticipation ability, where a time (or distance) between two consecutive measures, e.g. a finished vehicle braking measure and an output of positive gas measure (i.e. the driver gives gas), or a finished output of positive gas measure (i.e. the driver releases the gas) and a following vehicle braking measure, is measured. This measured time is compared with a reference parameter value representing the vehicle surrounding. Based on this comparison a value representing the driver's anticipation ability is determined.

The present invention relates to a braking anticipation ability determining system 18 for a vehicle. The system 18 includes an evaluation means to evaluate and grade a driver's braking anticipation ability, e.g. a device for determining a driver's anticipation ability, wherein the evaluation and grading depends on a measured time (or distance) t between a driver's release of gas measure and a following driver's braking measure. The driver's braking anticipation ability is determined in dependence of the result of a comparison of the time t and a reference value T representing at least one vehicle surrounding parameter, and at least one auxiliary brake value representing the use of the auxiliary brake. The present invention thus relates to a system for an extended evaluation and grading of a driver's braking anticipation ability, when the use of the auxiliary brakes is involved.

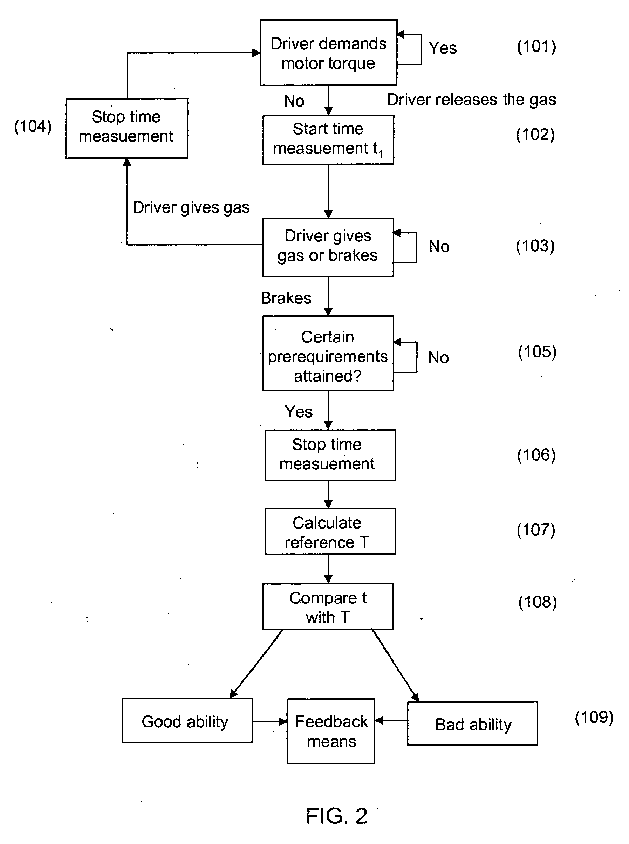

Fig. 2 shows a flow chart exemplifying an embodiment of the invention. The process begins in step 101, wherein the process remains for as long as the driver requests a positive motor torque (by pressing an accelerator pedal or manoeuvring another type of accelerator control). If a data processing unit included in the system 18 detects that the driver releases the accelerator pedal (i.e. the request for motor torque ceases), the process continues to step 102, where a time measurement is started, or alternatively, or in addition thereto, a distance measurement can be started, whereby the process continues to step 103 where it is determined whether the driver again requests a positive motor torque by pressing an accelerator pedal (or manoeuvring of another accelerator control), or if the driver requests a braking torque by activating any of the vehicle's brake systems, such as depressing a brake pedal or manoeuvring, e.g., a retarder control. The data processing unit included in the system 18 may consist of a processor, which is controlled by means of operation instructions, such as computer programs. For as long as the driver neither requests a motor torque nor a braking torque, the process remains in step 103 with activated time/distance measurement. However, if it is detected that the driver requests an acceleration the process continues to step 104 where time measurement (distance measurement) is stopped and the obtained time t (and/or distance s) is set to zero and the process returns to step 101. If it is detected that the driver brakes, then certain prerequirements in step 105 have to be attained to continue to step 106, where the time measurement is stopped. The certain prerequirements that should be attained are e.g. that an auxiliary torque value is sensed. After the time has been stopped in step 106, the process is continued to step 107 where it is calculated, or in other ways obtained, a reference value T representing a desired minimum time between request for acceleration and braking, which reference value T is determined based on the current traffic situation. The measured value is then, in step 108, compared to the reference value. If t > T, the driver's behavior is considered to show a good anticipation ability. If, however, t < T, the driver's behavior is considered to show a bad anticipation ability. Based on the comparison, a grade is given to the driver and may be presented to him/her on a presentation means (step 109), or sent to a distant fleet management system, for further evaluation and follow-up by e.g. haulers and other companies that use heavy vehicles in their business. The vehicle may also be arranged to continuously transmit data to a distant monitoring central, whereby the evaluation and grading may be performed in the monitoring central, instead of in the vehicle. Feedback may then be sent back to the vehicle to be presented to the driver.

The length of the reference time T can depend on the traffic situation. This can consist of traffic environment and speed of the vehicle. For example, the shortest desired reference time T can be obtained as:

$$T = f\ (surroundings)\ \cdot\ g(speed) + f(auxiliary\_brake) + k,$$

where $k$ is constant and $f\ (surroundings)$ constitute a function of the surroundings and represent the time slot demand of the surroundings. When driving on a freeway, a longer time slot is desired compared to an urban environment. $g(speed)$ is a function of the speed; $g(v) = c_1 \cdot v^b$, where $c_1$ and $b$ constitutes constants. $f(auxiliary\ brake)$ constitutes a function representing the time delay before an auxiliary torque is sensed when an auxiliary brake is activated.

The system 18 comprises means for receiving signals representing accelerator pedal positions and brake pedal positions. These signals can consist of a representation of the actual position of the pedals, which can be read using an appropriate sensor, or a calculated "position" based on a, by means of a measure taken by the driver, added brake torque or requested motor torque. For example, the position of a brake control, set by the driver, for a certain brake system can, by means of the construction of the vehicle, be arranged to be translated into an electronic signal. The system 18 may further comprise means for receiving signals representing control settings, and thereby the requested brake torque for each brake system, respectively, with regard to various kinds of driver actuated systems.

When a driver intends to brake the vehicle with the retarder, the retarder lever is pulled to an active position. Preferably, the system includes a retarder sensing means for sensing a retarder status value representing the status of the retarder as being in an active or an inactive state. This status signal indicates if e.g. a retarder lever is drawn or not, and may be represented as an electrical signal to the system 18.

It takes a certain time between that the driver draws the retarder bar until a braking effect is obtained, because of the physical properties of the retarder. Thus, the system 18 preferably includes a retarder torque sensing means for sensing a retarder torque value to determine when a braking effect is obtained. The system 18 calculates, as explained above, a time t between a driver's release of gas measure and a following driver's braking measure. To be counted as a driver's braking measure, the driver's braking measure has to, according to one embodiment, include that the retarder status value is in its active state, and that a retarder torque value above a certain threshold is sensed.

In one embodiment, the system 18 gives feedback to the driver of a preferred braking measure. This feedback encourages the driver to act in a preferred way. For example, if a driver has used the retarder before a crossing, there is a probability that the driver forgets to return the retarder lever to its place of origin, i.e. the driver forgets to inactivate the retarder. When the driver has finished his/her retardation and returns to control the speed with speed with help of the gas pedal, the retarder effect is automatically deactivated. If the driver subsequently releases the gas pedal the retarder starts a new retardation. To avoid this to happen, the system 18 may advantageously include a time measuring means for measuring a retarder time value. This retarder time value is started to be measured when the driver gives gas. If then an output of positive torque is measured, i.e. if the driver gives gas, if said retarder time value is above a certain time criterion and if the retarder status value is in its active state, then feedback is given to the driver to deactivate the retarder, i.e. release the retarder lever. This tip is thus shown before the driver has released the gas. If the tip is followed, the driver's anticipation capability is not evaluated and graded, because the driver has "saved" the situation.

The driver's braking anticipation ability is continuously graded, and is given as feedback to the driver. Advantageously, the system 18 gives feedback on a presentation means, but feedback may also be given by sound means. Feedback data from the system 18 may be continuously transmitted to a remote fleet management system, or being continuously presented to the vehicle driver. Driver trends may be registered to further evaluate the driver's driving manner. The retarder can be used manually by the driver to control the speed of the vehicle. It can also be set to operate automatically, according to the driver's needs. For example, in the fully automatic mode, a very short requested braking action (quick dab) on the service brake pedal engages a downhill speed control, including a constant speed brake function. This means that the exhaust brake is operated automatically whenever needed, in conjunction with the retarder, to control the vehicle's speed. This quick dab on the service brake pedal should not affect the driver's braking anticipation ability grade. In one embodiment, if a constant speed brake is activated, a driver's braking measure includes that an auxiliary brake torque value is sensed. Accordingly, to be counted as a driver's braking measure, the driver's braking measure has to include that a constant speed brake is activated and that a retarder or exhaust brake torque value above a certain threshold is sensed. The system 18 then preferably includes an exhaust brake torque sensing means for sensing an exhaust brake torque value, and means for determining a constant speed status signal indicating if the constant speed is activated or inactivated. By comparing the constant speed status signal with the short braking action, the intention to activate the downhill speed control can be secured. This status signal may be represented as an electrical signal to the system 18.

In general, motor braking should be rewarded. If the time t between a driver's release of gas measure and a following driver's braking measure is long, the grade given to the driver should be higher than general, to reward the driver for his/her braking anticipation ability. A prerequisite for evaluating and grading a motor braking is that the velocity is reduced a certain amount during the time the motor braking is carried out. The demanded velocity reduction is inter alia dependent on the load weight and velocity of the vehicle.

At low velocities, it is not demanded to use the auxiliary brakes, as it is not possible to brake to standstill with the auxiliary brakes because of the low brake torque the auxiliary brakes gives at low velocities. Thus, in one embodiment, a prerequisite for evaluating and grading the use of the auxiliary brakes, is that the maximum brake torque from the auxiliary brakes is above a certain threshold when the vehicle is driven with a velocity below a certain threshold (or when the rotational speed of the vehicle is below a certain threshold). This is the case for example when the vehicle is driven in a line of cars. Thus, no evaluation and grading is made of the use of the retarder, if the retarder is unable to activate because the velocity of the vehicle is below a certain threshold, as then no brake energy is retrieved from the retarder. The grades may be shown on an overview on the aforementioned presentation means.

An overall aim is to reduce the use of the brakes in a vehicle. If a driver can avoid braking, braked away energy can be saved. Feedback may thus be given as momentary fuel consumption versus mean consumption, to further encourage the driver to drive more economically.

The braking anticipation ability determining system 18 according to the invention could be used in other situations, e.g. to evaluate if the driver is in a stressed situation and grade the driver's braking anticipation ability accordingly. There are numerous ways to establish if a driver is in a stressed situation. It may be detected if the driver uses functions in the vehicle in a very fast manner, for example rapid use of the steering wheel. Another situation considered to be associated with stress is when the vehicle is exposed to high lateral acceleration (typically when driving in roundabouts) or when the driver activates the flashing indicators. If it is detected that a driver is in a stressed situation, the driver's braking anticipation ability is according to one embodiment graded, but no feedback concerning recommendations about preferred braking measures is given during the period with stress.

The present invention is not limited to the above-described preferred embodiments. Various alternatives, modifications and equivalents may be used. Therefore, the above embodiments should not be taken as limiting the scope of the invention, which is defined by the appending claims.

Examples

1. Braking anticipation ability determining system for a vehicle comprising: an evaluation means to evaluate and grade a driver's braking anticipation ability, wherein the evaluation and grading depends on a measured time t between a driver's release of gas measure and a following driver's braking measure, characterized in that the driver's braking anticipation ability is determined in dependence of the result of a comparison of said time t and a reference value T representing at least one vehicle surrounding parameter, and at least one auxiliary brake value representing the use of an auxiliary brake, and that the system gives feedback to the driver of a preferred braking measure.

2. Braking anticipation ability system according to example 1, wherein the system further comprises a retarder sensing means for sensing a retarder status value representing the status of the retarder as being in an active or an inactive state.

3. Braking anticipation ability system according to example 1 or 2, wherein the system further comprises a retarder torque sensing means for sensing a retarder torque value.

4. Braking anticipation ability system according to example 1, 2 or 3, wherein the system further comprises an exhaust brake torque sensing means for sensing an exhaust brake torque value.

5. Braking anticipation ability system according to example 2 and 3, wherein said driver's braking measure comprises that said retarder status value is in its active state, and that a retarder torque value above a certain threshold is sensed.

6. Braking anticipation ability system according to example 2 and 5, wherein the system further comprises a time measuring means for measuring a retarder time value, and
if said retarder status value is in its active state,
if an output of positive torque is measured, i.e. if the driver gives gas, and
if said retarder time value is above a certain time criterion, then feedback is given to the driver to release the retarder.

7. Braking anticipation ability system according to example 3 or 4, wherein if a constant speed brake is activated, said driver's braking measure comprises that an auxiliary brake torque value above a certain threshold is sensed.

8. Braking anticipation ability system according to any of the preceding examples, wherein the driver's braking anticipation ability is continuously graded, and is given as feedback to the driver.

9. Braking anticipation ability system according to any of the preceding examples, wherein the system gives feedback on a presentation means.

10. Vehicle comprising a braking anticipation ability system according to any of the preceding examples.

**Claims**

1. Braking anticipation ability determining system (18) for a vehicle comprising:

   an evaluation means to evaluate and grade a driver's braking anticipation ability; and
   a retarder sensing means for sensing a retarder status value representing the status of the retarder as being in an active or an inactive state;

   **characterized in that** if said retarder status value is in its active state and if the driver gives gas, then feedback is given to the driver to release the retarder.

2. The system (18) according to claim 1, further comprising:

   a time measuring means for measuring a retarder time value;

   wherein if said retarder status value is in its active state, if the driver gives gas, and if said retarder time value is above a certain time criterion, then feedback is given to the driver to release the retarder.

3. The system (18) according to claim 1 or 2, wherein the system is configured to determine if the driver gives gas by measuring an output of positive torque.

4. The system (18) according to claim 2, wherein the system is configured to start measuring the retarder time value when the driver gives gas.

5. The system (18) according to any preceding claim, further comprising a retarder torque sensing means for sensing a retarder torque value.

6. The system (18) according to claim 5, wherein the system is configured to use the retarder torque value to determine when a braking effect is obtained.

7. The system (18) according to any preceding claim, wherein the system is configured to give feedback on a presentation means.

8. The system (18) according to any preceding claim, wherein the system is configured to continuously transmit feedback data from the system (18) to a remote fleet management system.

9. The system (18) according to any preceding claim, wherein the evaluation means is configured to not evaluate and grade the use of the retarder when the velocity of the vehicle is below a certain threshold.

10. The system (18) according to any preceding claim, wherein the system is configured to evaluate if the driver is in a stressed situation and if it is detected that a driver is in a stressed situation, the driver's braking anticipation ability is graded, but no feedback concerning recommendations about preferred braking measures is given during the period with stress.

11. Vehicle comprising a braking anticipation ability system (18) according to any preceding claim.

12. The vehicle according to claim 11, wherein the vehicle is configured to automatically deactivate the retarder effect when the driver has finished a retardation and returns to control the speed with a gas pedal.


**Patentansprüche**

1. System (18) zum Bestimmen der Fähigkeit zum Antizipieren eines Bremsvorgangs für ein Fahrzeug, umfassend:

    ein Bewertungsmittel zum Bewerten und Einstufen einer Fähigkeit eines Fahrers zum Antizipieren eines Bremsvorgangs; und
    ein Retardererfassungsmittel zum Erfassen eines Retarderzustandswertes, der den Zustand des Retarders als sich in einem aktiven oder inaktiven Zustand befindlich darstellt;

    **dadurch gekennzeichnet, dass** dann, wenn der Retarderzustandswert in seinem aktiven Zustand ist und der Fahrer Gas gibt, eine Rückmeldung an den Fahrer gegeben wird, den Retarder zu lösen.

2. System (18) gemäß Anspruch 1, ferner umfassend:

    ein Zeitmessmittel zum Messen eines Retarderzeitwertes;

    wobei dann, wenn der Retarderzustandswert in seinem aktiven Zustand ist, wenn der Fahrer Gas gibt und wenn der Retarderzeitwert oberhalb eines bestimmten Zeitkriteriums liegt, eine Rückmeldung an den Fahrer gegeben wird, den Retarder zu lösen.

3. System (18) gemäß Anspruch 1 oder 2,
    wobei das System dazu eingerichtet ist, durch Messen einer Abgabe eines positiven Drehmoments zu ermitteln, ob der Fahrer Gas gibt.

**4.** System (18) gemäß Anspruch 2,
wobei das System dazu eingerichtet ist, das Messen des Retarderzeitwertes zu starten, wenn der Fahrer Gas gibt.

**5.** System (18) gemäß einem der vorangehenden Ansprüche,
ferner umfassend ein Retarderdrehmomenterfassungsmittel zum Erfassen eines Retarderdrehmomentwertes.

**6.** System (18) gemäß Anspruch 5,
wobei das System dazu eingerichtet ist, den Retarderdrehmomentwert zu verwenden, um zu ermitteln, wann eine Bremswirkung erzielt wird.

**7.** System (18) gemäß einem der vorangehenden Ansprüche,
wobei das System dazu eingerichtet ist, auf einem Darstellungsmittel Rückmeldung zu geben.

**8.** System (18) gemäß einem der vorangehenden Ansprüche,
wobei das System dazu eingerichtet ist, kontinuierlich Rückmeldungsdaten von dem System an ein entferntes Flottenmanagementsystem zu übertragen.

**9.** System (18) gemäß einem der vorangehenden Ansprüche,
wobei das Bewertungsmittel dazu eingerichtet ist, das Benutzen des Retarders nicht zu bewerten und einzustufen, wenn die Geschwindigkeit des Fahrzeugs unterhalb einer bestimmten Schwelle liegt.

**10.** System (18) gemäß einem der vorangehenden Ansprüche,
wobei das System dazu eingerichtet ist, zu bewerten, ob sich der Fahrer in einer gestressten Situation befindet, und dann, wenn ermittelt wird, dass sich der Fahrer in einer gestressten Situation befindet, wird die Fähigkeit des Fahrers zum Antizipieren eines Bremsvorgangs eingestuft, aber während der Zeitdauer mit Stress wird keine Rückmeldung bezüglich Empfehlungen zu bevorzugten Bremsmitteln gegeben.

**11.** Fahrzeug,
umfassend ein System (18) zum Bestimmen der Fähigkeit zum Antizipieren eines Bremsvorgangs gemäß einem der vorangehenden Ansprüche.

**12.** Fahrzeug gemäß Anspruch 11,
wobei das Fahrzeug dazu eingerichtet ist, automatisch die Retarderwirkung zu deaktivieren, wenn der Fahrer eine Abbremsung beendet hat und dazu zurückkehrt, die Geschwindigkeit mit einem Gaspedal zu steuern.

**Revendications**

**1.** Système (18) de détermination d'aptitude à l'anticipation de freinage pour un véhicule, comprenant :

des moyens d'évaluation pour évaluer et noter une aptitude à l'anticipation de freinage d'un conducteur ; et des moyens de détection de ralentisseur pour détecter une valeur d'état de ralentisseur représentant l'état du ralentisseur, à savoir étant dans un état actif ou inactif ;
**caractérisé en ce que**, si ladite valeur d'état de ralentisseur est dans son état actif et si le conducteur effectue une accélération, alors une rétroaction est délivrée au conducteur pour relâcher le ralentisseur.

**2.** Système (18) selon la revendication 1, comprenant en outre :

des moyens de mesure de temps pour mesurer une valeur de temps de ralentisseur ;
dans lequel, si ladite valeur d'état de ralentisseur est dans son état actif, si le conducteur effectue une accélération, et si ladite valeur de temps de ralentisseur est supérieure à un certain critère de temps, alors une rétroaction est délivrée au conducteur pour relâcher le ralentisseur.

**3.** Système (18) selon la revendication 1 ou 2, dans lequel le système est configuré de façon à déterminer si le conducteur effectue une accélération par la mesure d'une sortie de couple positif.

**4.** Système (18) selon la revendication 2, dans lequel le système est configuré de façon à débuter la mesure de la valeur de temps de ralentisseur lorsque le conducteur effectue une accélération.

**5.** Système (18) selon une quelconque revendication précédente, comprenant en outre des moyens de détection de couple de ralentisseur pour détecter une valeur de couple de ralentisseur.

**6.** Système (18) selon la revendication 5, dans lequel le système est configuré de façon à utiliser la valeur de couple de ralentisseur afin de déterminer le moment où un effet de freinage est obtenu.

**7.** Système (18) selon une quelconque revendication précédente, dans lequel le système est configuré de façon à délivrer une rétroaction sur des moyens de présentation.

**8.** Système (18) selon une quelconque revendication précédente, dans lequel le système est configuré de façon à transmettre de façon continue des données de rétroaction à partir du système (18) à un système de gestion de flotte à distance.

**9.** Système (18) selon une quelconque revendication précédente, dans lequel les moyens d'évaluation sont configurés de façon à ne pas évaluer et à ne pas noter l'utilisation du ralentisseur lorsque la vitesse du véhicule est inférieure à un certain seuil.

**10.** Système (18) selon une quelconque revendication précédente, dans lequel le système est configuré de façon à évaluer si le conducteur est dans une situation de stress, et, s'il est détecté qu'un conducteur est dans une situation de stress, l'aptitude à l'anticipation de freinage du conducteur est notée, mais aucune rétroaction concernant des recommandations au sujet de mesures de freinage préférées n'est délivrée durant la période sous stress.

**11.** Véhicule comprenant un système (18) d'aptitude à l'anticipation de freinage selon une quelconque revendication précédente.

**12.** Véhicule selon la revendication 11, dans lequel le véhicule est configuré de façon à désactiver automatiquement l'effet du ralentisseur lorsque le conducteur a achevé un ralentissement et revient à la commande de la vitesse avec une pédale d'accélérateur.

FIG. 1

Driver demands
motor torque ← Yes (101)

No | Driver releases the gas

(104) Stop time
measuement | Start time
measuement $t_1$ (102)

Driver gives gas

Driver gives
gas or brakes ← No (103)

Brakes

Certain
prerequirements
attained? ← No (105)

Yes

Stop time
measuement (106)

Calculate
reference T (107)

Compare t
with T (108)

Good ability → Feedback
means ← Bad ability (109)

FIG. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2007139493 A
- WO 2007133987 A
- WO 200713949 A1
- WO 0233529 A2
- US 6092021 A
- WO 0007150 A1
- WO 03020562 A1
- DE 19860248 C1
- US 2008255722 A